# FASCICULE DE BREVET EUROPEEN

(11) **EP 0 501 894 B1**
(45) Date de publication et mention de la délivrance du brevet: **09.10.1996**
(21) Numéro de dépôt: 92420033.0
(22) Date de dépôt: 28.01.1992
(51) Int. Cl.: B01D 19/00, A61M 1/16

(54) **Dispositif pour éliminer les bulles de gaz d'un liquide en circulation**
Vorrichtung zur Entfernung von Gasblasen aus einer zirkulierenden Flüssigkeit
Apparatus for eliminating gas bubbles from a circulating liquid

(30) Priorité: 01.03.1991 FR 9102692
(43) Date de publication de la demande: 02.09.1992
(73) Titulaire: HOSPAL INDUSTRIE, F-69883 Meyzieu Cédex (FR)
(72) Inventeur: Chevallet, Jacques, F-69360 Serezin du Rhone (FR); Cortial, Jean-Louis, F-69003 Lyon (FR)

(56) Documents cités:
- DE-C- 224 106
- FR-A- 1 262 910
- FR-A- 2 550 451
- GB-A- 451 905
- US-A- 4 976 754
- SOVIET PATENTS ABSTRACTS Week 8502, 20 Février 1985 Derwent Publications Ltd., London, GB; AN 85-011079/02

## Description

La présente invention concerne un dispositif pour éliminer les bulles de gaz d'un liquide en circulation, appelé couramment piège à bulles.

Les pièges à bulles sont utilisés dans des secteurs techniques variés comme composants des circuits hydrauliques où la présence de bulles de gaz est indésirable. A titre d'exemple, le circuit hydraulique des appareils utilisés en dialyse rénale pour fabriquer et faire circuler un liquide de dialyse dans un échangeur à membrane comprend plusieurs pièges à bulles sans lesquels le taux de transfert dans l'échangeur serait moindre, le fonctionnement des pompes pertubé et la mesure de la quantité de plasma sanguin ultrafiltrant, le cas échéant, à travers la membrane, faussée.

Un piège à bulles utilisé dans ce type d'appareil comprend, de façon classique, une cuve faiblement tronconique fermée par un couvercle présentant un bombement, au point haut duquel est connectée une canalisation de purge obturable. La cuve est séparée en deux sur une partie de sa hauteur par une cloison transversale, l'entrée et la sortie du liquide se faisant respectivement de part et d'autre de la cloison. Cette cloison a pour fonction d'empêcher le liquide entrant dans le piège à bulles d'en ressortir aussitôt. Le piège à bulles est muni à sa partie supérieure d'un détecteur de liquide qui est utilisé pour commander l'ouverture de la canalisation de purge chaque fois que ce détecteur ne détecte plus la présence de liquide. A titre indicatif, un piège à bulles de ce type ayant un volume interne d'environ 150 millilitres permet d'extraire en une minute environ 4,5 millilitres d'air (mesurés à la pression atmosphérique) d'un liquide circulant à un débit d'environ 1200 millilitres/minutes.

Le piège à bulles classique décrit ci-dessus présente plusieurs inconvénients. Compte tenu de sa forme, le volume d'air évacué à chaque purge est relativement important ce qui provoque une perturbation de l'écoulement du liquide, en particulier à l'entrée et à la sortie du piège à bulles. En outre, ce piège à bulles présente, par rapport aux canalisations tubulaires du circuit, un volume non négligeable, d'où une mise en route de l'appareil (stérilisation, remplissage initial, réchauffage) assez longue. Enfin, ce piège à bulles ne peut jamais être vidangé complètement.

Il est connu par ailleurs, par le document US 4 493 705, un réservoir à sang constitué par une poche souple en forme de banane ayant une entrée et une sortie s'étendant dans le prolongement de l'axe médian de la poche, ainsi qu'un évent situé au point haut de la poche, approximativement à mi distance entre l'entrée et la sortie. Bien que la forme de ce réservoir provoque un ralentissement du fluide y circulant dans sa partie centrale, elle n'est pas étudiée pour en faire disparaître les remous ni pour favoriser le dégagement des bulles hors du liquide en circulation. D'autre part, la vidange de ce réservoir requiert une intervention manuelle.

Un but de la présente invention est de réaliser un piège à bulles offrant des performances optimales tout en étant de volume réduit, et qui puisse être vidangé en totalité.

C'est pourquoi on prévoit, conformément à l'invention, un dispositif pour éliminer les bulles de gaz d'un liquide comprenant des moyens de canalisation pour le liquide ayant un axe central présentant une courbure au niveau d'un point haut pour le rassemblement des bulles par gravité, les moyens de canalisation ayant :
- une portion de section croissante en amont du point haut,
- une portion de section décroissante en aval du point haut ayant un axe central incliné par rapport à l'horizontale, la portion de section croissante étant située en amont de la section de portion décroissante par rapport au sens d'écoulement d'un liquide à dégazer, et
- des moyens pour provoquer la concentration dynamique des bulles en amont de la portion de section croissante des moyens de canalisation, comprenant une portion de canalisation courbe.

Dans un mode de réalisation préféré, les moyens de concentration dynamique des bulles comprennent une portion de canalisation courbe située en amont de la portion de canalisation de section croissante, cette portion de canalisation courbe ayant une section sensiblement constante et un rayon de courbure inférieur à celui des moyens de canalisation au niveau du point haut. Elle définit une trajectoire courbe extérieure pour les bulles se raccordant à un trajet de plus grande pente supérieure des moyens de canalisation, aboutissant au point haut.

Cette disposition favorise considérablement l'élimination des bulles présentes dans le liquide entrant dans le piège à bulles, par un effet d'écoulement dynamique qui tend à rassembler et à chasser les bulles vers l'extérieur du tronçon courbe, et vient s'ajouter à la décantation par gravité. Ainsi, il est possible de réaliser des pièges à bulles, qui, pour un même rendement, ont un volume très inférieur à celui des pièges à bulles classiques.

Selon une caractéristique de l'invention, le dispositif comprend des moyens de recueil pour le gaz constitués par une alvéole oblongue ouvrant sur les moyens de canalisation, au niveau du point haut.

Cette disposition permet de n'éliminer à chaque purge qu'une faible quantité de gaz, de sorte que la circulation du liquide dans les moyens de canalisation ne s'en trouve pas perturbée.

Selon une autre caractéristique de l'invention, le dispositif comprend en outre des moyens pour provoquer un écoulement sensiblement sans remous du liquide dans les moyens de canalisation. Ces moyens comprennent un profil des moyens de canalisation configuré pour épouser la veine liquide en sortie du tronçon courbe ainsi que, à la jonction des moyens de canalisation et de l'alvéole pour le recueil des bulles, une cloison assurant la continuité des moyens de canalisation, munie d'au moins une fente pour le passage des bulles.

Grâce à cette disposition, les bulles regroupées dans le tronçon courbe ne sont pas dissociées ultérieurement et l'action de la gravité sur les bulles n'est pas contrariée.

Selon encore une autre caractéristique de l'invention, la ligne de niveau inférieure des moyens de canalisation, considérée dans le sens de l'écoulement du liquide, a une pente sensiblement négative ou nulle à partir de l'extrémité aval de la portion de canalisation courbe.

Grâce à cette disposition, le piège à bulles selon l'invention peut être vidangé quasi complètement par gravité.

D'autres caractéristiques et avantages de la présente invention apparaîtront à la lecture de la description qui va suivre. On se reportera aux dessins annexés sur lesquels :
La figure 1 est une vue en coupe selon un plan horizontal d'un mode de réalisation du piège à bulles selon l'invention ; et.
la figure 2 est un diagramme de la vitesse moyenne de l'écoulement d'un liquide dans le piège à bulles de la figure 1.

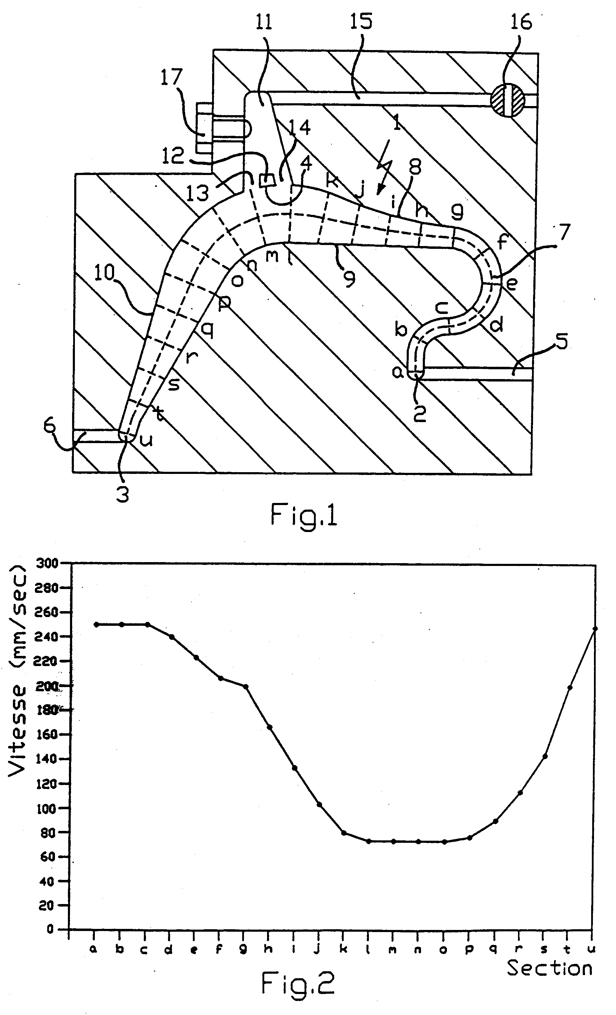

Le piège à bulles de la figure 1 comporte des moyens de canalisation pour le liquide à dégazer constitués par un canal de section variable 1 ayant une entrée 2 et une sortie 3 disposées au-dessous du niveau d'un tronçon de décantation intermédiaire comprenant un point haut 4 pour le rassemblement des bulles par gravité. L'entrée 2 et la sortie 3 sont respectivement reliées à des canaux 5, 6 destinés à être connectés à des tubulures d'un circuit hydraulique. Le canal 1 présente une courbure au niveau du point haut 4.

Conformément à l'invention, le canal 1 comprend, en amont du point haut 4, un tronçon courbe 7 ayant sa convexité tournée vers l'extérieur, formant des moyens pour provoquer la concentration dynamique des bulles. Le tronçon 7 a un rayon de courbure inférieur à celui du canal 1 au niveau du point haut 4. Ce tronçon courbe a une section sensiblement constante et il se raccorde au tronçon de décantation sans rupture de continuité, la courbe extérieure du tronçon courbe se prolongeant par la ligne de plus grande pente supérieure 8, aboutissant au point haut 4, du tronçon de décantation. A partir de l'extrémité aval du tronçon courbe 7, la section du canal 1 croît progressivement pour devenir sensiblement constante de part et d'autre du point haut 4, puis elle décroît progressivement jusqu'à la sortie 3, qui est située au-dessous du niveau de l'entrée 2 et présente une section sensiblement égale à celle de l'entrée 2. Le canal 1 est profilé pour épouser la veine de liquide en sortie du tronçon courbe 7, d'où notamment la légère convexité, tournée vers l'intérieur du canal 1, de la ligne de plus grande pente supérieure 8, entre l'extrémité aval du tronçon courbe 7 et le point haut 4. Considérée dans le sens de l'écoulement du liquide, la ligne de niveau inférieur 9 du canal 1 a, à partir de l'extrémité aval du tronçon courbe 7, une pente sensiblement nulle jusqu'à l'aplomb du point haut 4 puis négative jusqu'à la sortie 3. L'angle que fait la ligne de plus grande pente supérieure 10 du tronçon terminal du canal 1 est choisi pour que les bulles qui pourraient se déposer sur la paroi correspondante puissent remonter par gravité le long de cette paroi jusqu'au point haut 4 sans risquer de se décoller ni d'être entraînées par le liquide. Avantageusement, cet angle est compris entre environ 20° et environ 30° par rapport à la verticale.

Des moyens de recueil de gaz constitués par une alvéole oblongue 11 d'axe sensiblement perpendiculaire à l'axe du canal 1, ouvrent sur le tronçon de décantation en une zone centrée approximativement sur le point haut 4 du canal 1. Pour éviter qu'il ne se produise de remous au niveau de la jonction de l'alvéole 11 et du canal 1, le débouché de l'alvéole 11 est partiellement barré par une cloison 12 dont la surface inférieure s'étend dans la continuité de la paroi supérieure du canal 1 et qui délimite deux fentes 13, 14 pour le passage des bulles.

L'alvéole 11 est équipée de moyens de purge constitués par une canalisation 15, oburable par une vanne 16, reliant la partie haute de l'alvéole à l'atmosphère. La vanne 16 est commandée à partir des informations fournies par un détecteur de liquide 17 disposé pour intercepter l'alvéole 11, à mi-hauteur.

Sur le diagramme de la figure 2, on voit que, pour un débit d'entrée constant, la vitesse du liquide ne décroît d'abord que lentement de l'entrée 2 (section a) à l'extrémité aval du tronçon courbe 7 (section g) (perte de vitesse de environ 20 %), puis rapidement de la section g à la section k, à partir de laquelle elle se stabilise à environ un tiers de la vitesse d'entrée dans la portion du canal 1 de section maximale et constante (sections k à p), pour croître ensuite rapidement à partir de la section q et reprendre, à la sortie 3 (section u) du piège à bulles, la valeur qu'elle avait à l'entrée. On observe que les bulles présentes dans le liquide entrant dans le piège à bulles sont chassées massivement vers la paroi extérieure du tronçon courbe 7, à la sortie duquel elles restent plaquées contre la paroi supérieure du canal 1 et glissent en direction de l'alvéole 11 sous l'effet de la gravité et de l'écoulement du liquide. Comme la veine liquide est dépourvue de remous, les bulles regroupées dans le tronçon courbe le restent. Par ailleurs, grâce aussi à l'absence de remous, les bulles qui seraient restées à l'intérieur de la veine liquide décantent par gravité dans le tronçon de décantation où la vitesse du liquide est relativement faible. Elles s'accumulent contre la paroi supérieure du canal 1, de part et d'autre de l'ouverture de l'alvéole 11 en direction de laquelle elles glissent (sous le seul effet de la gravité pour les bulles en aval de l'alvéole 11) pour finir par s'y accumuler. Au fur et à mesure que l'alvéole 11 se remplit de gaz, le niveau du liquide dans l'alvéole 11 baisse jusqu'à ce que le détecteur 17 ne détecte plus la présence de liquide. La vanne 16 est alors ouverte, le gaz présent dans l'alvéole 11 est purgé, le niveau de liquide remonte dans l'alvéole et la vanne est refermée. Compte tenu de la forme de l'alvéole 11, dont la section est réduite, la quantité de gaz éliminé à chaque purge est faible et les purges fréquentes. L'écoulement du liquide dans le piège à bulles n'en est pas pertubé.

Un piège à bulles, comme celui qui est représenté à l'échelle 1 sur la figure 1, avec un canal 1 ayant une section droite rectangulaire et une largeur de 2,5 centimètres, a un volume d'environ 40 millilitres, soit environ un quart du volume d'un piège à bulles classique. Pour un débit de liquide d'environ 1200 millilitres/minute, il permet d'extraire du liquide environ 4,5 millilitres de gaz (mesurés à la pression atmosphérique) en une minute, c'est-à-dire la même quantité de gaz qu'un piège à bulles classique.

En envoyant un gaz sous pression dans le piège à bulles 1 rempli de liquide, par la canalisation 5, il est possible de vidanger totalement ce piège à bulles.

La présente invention n'est pas limitée au mode de réalisation qui vient d'être décrit et elle est susceptible de variantes.

## Revendications

1. Dispositif pour éliminer les bulles de gaz d'un liquide comprenant des moyens de canalisation (1) pour le liquide ayant un axe central présentant une courbure au niveau d'un point haut (4) pour le rassemblement des bulles par gravité, les moyens de canalisation (1) ayant :
- une portion de section croissante en amont du point haut (4),
- une portion de section décroissante en aval du point haut (4) ayant un axe central incliné par rapport à l'horizontale, la portion de section croissante étant située en amont de la section de portion décroissante par rapport au sens d'écoulement d'un liquide à dégazer, et
- des moyens (7) pour provoquer la concentration dynamique des bulles en amont de la portion de section croissante des moyens de canalisation, comprenant une portion de canalisation courbe (7).

2. Dispositif selon la revendication 1, caractérisé en ce que la concavité de la portion de canalisation courbe (7) et la concavité de l'axe central de la courbure des moyens de canalisation (1) sont orientées vers une même zone.

3. Dispositif selon une des revendications 1 et 2, caractérisé en ce que la portion de canalisation courbe (7) définit une trajectoire courbe extérieure se raccordant à un trajet de plus grande pente supérieure (8) de la portion de section croissante des moyens de canalisation (1).

4. Dispositif selon une des revendications 1 à 3, caractérisé en ce qu'il comprend des moyens de recueil pour le gaz constitués par une alvéole oblongue (11) ouvrant sur les moyens de canalisation (1), au niveau du point haut (4).

5. Dispositif selon une des revendications 1 à 4, caractérisé en ce qu'il comprend en outre des moyens (12) pour provoquer un écoulement sensiblement sans remous du liquide dans les moyens de canalisation (1).

6. Dispositif selon les revendications 4 et 5, caractérisé en ce que les moyens pour provoquer un écoulement sensiblement sans remous du liquide dans les moyens de canalisation (1) comprennent, à la jonction des moyens de canalisation (1) et de l'alvéole (11) pour le recueil des bulles, une cloison (12) assurant la continuité des moyens de canalisation (1), munie d'au moins une fente (13, 14) pour le passage des bulles.

7. Dispositif selon une des revendications 5 et 6, caractérisé en ce que les moyens pour provoquer un écoulement sensiblement sans remous du liquide dans les moyens de canalisation (1), comprennent un profil des moyens de canalisation (1) configuré pour épouser la veine liquide en sortie de la portion de canalisation courbe (7).

8. Dispositif selon une des revendications 1 à 7, caractérisé en ce que la ligne de niveau inférieure (9) de la portion de section croissante des moyens de canalisation (1), considérée dans le sens de l'écoulement du liquide, a une pente sensiblement négative ou nulle à partir de l'extrémité aval de la portion de canalisation courbe (7).

9. Dispositif selon une des revendications 1 à 8, caractérisé en ce que l'angle que fait la ligne de plus grande pente supérieure (10) de la portion de section décroissante des moyens de canalisation (1) est compris entre environ 20° et environ 30° par rapport à la verticale.

10. Dispositif selon une des revendications 1 à 9, caractérisé en ce que les moyens de canalisation (1) ont, au niveau de leur entrée (2) et de leur sortie (3), des sections sensiblement égales.

11. Dispositif selon une des revendications 1 à 10, caractérisé en ce que la sortie (3) des moyens de canalisation (1) est située à un niveau inférieur au niveau de leur entrée (2).

12. Dispositif selon une des revendications 4 à 11, caractérisé en ce qu'il comprend des moyens de purge comportant un canal obturable (15, 16) reliant le point haut des moyens de recueil (11) à l'atmosphère.

13. Dispositif selon la revendication 12, caractérisé en ce qu'il comprend un détecteur de présence de liquide (17) pour détecter la présence de liquide dans les moyens de recueil (11), les moyens de purge (15, 16) étant actionnés lorsque le détecteur (17) ne détecte plus la présence de liquide.

14. Dispositif selon une des revendications 1 à 13, caractérisé en ce qu'il a un volume d'environ 40.10⁻³ litres et en ce qu'il permet d'extraire environ 4,5.10⁻³ litres de gaz par minute d'un liquide circulant à environ 1,2 litres/minute.

## Claims

1. A device for eliminating bubbles of gas from a liquid, the device comprising duct means (1) for the liquid and having a central axis curved at a high point (4) to enable bubbles to collect under gravity, the duct means having :
- a portion of flaring section upstream from the high point (4),
- a portion of tapering section downstream from the high point (4), having a central axis inclined with respect to the horizontal, the portion of flaring section being situated upstream from the portion of tapering section with respect to the direction of flow of a liquid to be degassed, and
- means (7) for dynamically concentrating the bubbles upstream from the portion of flaring section of the duct means, comprising a curved duct portion (7).

2. A device according to claim 1, characterized in that the concavity of the curved duct portion (7) and the concavity of the central axis of the curve of the duct means (1) face a same area.

3. A device according to one of claims 1 and 2, characterized in that the curved duct portion (7) defines an outer curved trajectory running into a top path of greatest slope (8) of the portion of flaring section of the duct means.

4. A device according to one of claims 1 to 3, characterized in that it comprises gas collection means constituted by an oblong cell (11) opening out to the duct means (1) at the high point (4).

5. A device according to one of claims 1 to 4, characterized in that it further comprises means (12) for causing the liquid to flow through the duct means substantially without eddies.

6. A device according to claims 4 and 5, characterized in that the means for causing the liquid to flow through the duct means (1) substantially without eddies comprises a partition (12) at the junction between the duct means (1) and the cell (11) for collecting the bubbles, which partition (12) provides continuity for the duct means and is provided with at least one slot (13, 14) for passing bubbles.

7. A device according to one of claims 5 and 6, characterized in that the means for causing the liquid to flow through the duct means (1) substantially without eddies comprise a profile for the duct means (1) shaped so as to follow the liquid streamlines leaving the curved duct portion (7).

8. A device according to one of claims 1 to 7, characterized in that the bottom level line (9) of the portion of flaring section of the duct means (1) has a slope in the liquid flow direction which is substantially negative or zero starting from the downstream end of the curved duct portion (7).

9. A device according to one of claims 1 to 8, characterized in that the angle, made by the top line of greatest slope (10) of the portion of tapering section of the duct means (1), lies in the range of about 20° to about 30° with respect to the vertical.

10. A device according to one of claims 1 to 9, characterized in that the duct means (1) are of substantially equal section at their inlet (2) and at their outlet (3).

11. A device according to one of claims 1 to 10, characterized in that the outlet (3) of the duct means (1) is situated at a lower level than the inlet thereto (2).

12. A device according to one of claims 4 to 11, characterized in that it comprises purge means (15, 16) including a closable channel connecting the high point of the collection means (11) to the atmosphere.

13. A device according to claim 12, characterized in that it comprises a liquid presence detector (17) for detecting the presence of liquid in the collection means (11), with the purge means (15, 16) being actuated when the detector (17) no longer detects the presence of liquid.

14. A device according to one of claims 1 to 13, characterized in that it has a volume of about 40.10⁻³ liters and enables about 4.5.10⁻³ liters of gas to be extracted from the liquid per minute, with a liquid flow rate of about 1,2 liter per minute.

## Patentansprüche

1. Vorrichtung zur Entfernung der Gasblasen aus einer Flüssigkeit, die Leitungsmittel (1) für die Flüssigkeit umfaßt, die eine zentrale Achse mit einer Krümmung an einer hohen Stelle (4) zur von der Schwerkraft bewirkten Sammlung der Blasen aufweisen, wobei die Leitungsmittel (1) folgendes aufweisen:
- einen Teil mit zunehmendem Querschnitt vor der hohen Stelle (4),
- einen Teil mit abnehmendem Querschnitt hinter der hohen Stelle (4) mit einer bezüglich der Horizontalen geneigten zentralen Achse, wobei der Teil mit zunehmendem Querschnitt, in Fließrichtung einer zu entgasenden Flüssigkeit gesehen, vor dem Teil mit abnehmendem Querschnitt angeordnet ist, und
- einen gekrümmten Leitungsteil (7) umfassende Mittel (7) zum Bewirken, daß sich die Blasen dynamisch vor dem Teil mit zunehmendem Querschnitt der Leitungsmittel konzentrieren.

2. Vorrichtung nach Anspruch 1, dadurch gekennzeichnet, daß die Rundhöhlung des gekrümmten Leitungsteils (7) und die Rundhöhlung der zentralen Achse der Krümmung der Leitungsmittel (1) auf dieselbe Zone gerichtet sind.

3. Vorrichtung nach Anspruch 1 oder 2, dadurch gekennzeichnet, daß der gekrümmte Leitungsteil (7) eine gekrümmte äußere Bahn definiert, die an eine Bahn mit größerem Obergefälle (8) des Teils mit zunehmendem Querschnitt der Leitungsmittel (1) anschließt.

4. Vorrichtung nach einem der Ansprüche 1 bis 3, dadurch gekennzeichnet, daß sie Aufnahmemittel für das Gas umfaßt, die aus einer länglichen Zelle (11) bestehen, die an der hohen Stelle (4) in die Leitungsmittel (1) mündet.

5. Vorrichtung nach einem der Ansprüche 1 bis 4, dadurch gekennzeichnet, daß sie weiterhin Mittel (12) umfaßt, die ein im wesentlichen strudelfreies Fließen der Flüssigkeit in den Leitungsmitteln (1) hervorrufen.

6. Vorrichtung nach Ansprüchen 4 und 5, dadurch gekennzeichnet, daß die Mittel, die ein im wesentlichen strudelfreies Fließen der Flüssigkeit in den Leitungsmitteln (1) hervorrufen, an dem Schnittpunkt der Leitungsmittel (1) und der Zelle (11) zur Aufnahme der Blasen eine die Kontinuität der Leitungsmittel (1) gewährleistende Trennwand (12) umfassen, die mindestens einen Spalt (13, 14) für den Blasendurchgang aufweist.

7. Vorrichtung nach Anspruch 5 oder 6, dadurch gekennzeichnet, daß die Mittel, die ein im wesentlichen strudelfreies Fließen der Flüssigkeit in den Leitungsmitteln (1) hervorrufen, ein Profil der Leitungsmittel (1) umfassen, das so ausgelegt ist, daß es sich an den aus dem gekrümmten Leitungsteil (7) austretenden Flüssigkeitsstrom anschmiegt.

8. Vorrichtung nach einem der Ansprüche 1 bis 7, dadurch gekennzeichnet, daß die untere Niveaukurve (9) des Teils mit zunehmendem Querschnitt der Leitungsmittel (1), in Fließrichtung der Flüssigkeit gesehen, ab dem hinteren Ende des gekrümmten Leitungsteils (7) eine im wesentlichen negative Neigung oder eine Neigung von null hat.

9. Vorrichtung nach einem der Ansprüche 1 bis 8, dadurch gekennzeichnet, daß der Winkel, den die Linie mit größerem Obergefälle (10) des Teils mit abnehmendem Querschnitt der Leitungsmittel (1) einschließt, etwa 20° bis etwa 30° zur Vertikalen beträgt.

10. Vorrichtung nach einem der Ansprüche 1 bis 9, dadurch gekennzeichnet, daß die Leitungsmittel (1) auf der Höhe ihres Eingangs (2) und ihres Ausgangs (3) im wesentliche gleiche Querschnitte haben.

11. Vorrichtung nach einem der Ansprüche 1 bis 10, dadurch gekennzeichnet, daß der Ausgang (3) der Leitungsmittel (1) niedriger liegt als ihr Eingang (2).

12. Vorrichtung nach einem der Ansprüche 4 bis 11, dadurch gekennzeichnet, daß sie Ablaßmittel umfaßt, die aus einem verschließbaren Kanal (15, 16) bestehen, der die hohen Stelle der Aufnahmemittel (11) mit der Atmosphäre verbindet.

13. Vorrichtung nach Anspruch 12, dadurch gekennzeichnet, daß sie einen Flüssigkeitsdetektor (17) umfaßt, der das Vorliegen von Flüssigkeit in den Aufnahmemitteln (11) erfaßt, wobei die Ablaßmittel (15, 16) dann betätigt werden, wenn der Detektor (17) erfaßt, daß keine Flüssigkeit mehr vorliegt.

14. Vorrichtung nach einem der Ansprüche 1 bis 13, dadurch gekennzeichnet, daß sie ein Fassungsvermögen von etwa 40.10⁻³ Litern hat und daß man ungefähr 4,5.10⁻³ Liter Gas pro Minute aus einer mit einem Durchsatz von etwa 1,2 Litern/Minute zirkulierenden Flüssigkeit abziehen kann.
